(19) Europäisches Patentamt
European Patent Office
Office européen des brevets

(11) Veröffentlichungsnummer: **0 013 660**
**B1**

(12) **EUROPÄISCHE PATENTSCHRIFT**

(45) Veröffentlichungstag der Patentschrift: **18.05.83**

(21) Anmeldenummer: **80810002.8**

(22) Anmeldetag: **04.01.80**

(51) Int. Cl.³: **C 07 C 93/20**, C 07 D 295/08, C 07 C 131/00, C 07 D 213/30, C 07 D 207/24, C 07 D 211/22, C 07 D 211/46, A 01 N 37/18, A 01 N 43/36, A 01 N 43/40

(54) **Aminoalkylester der 2-Nitro-5-(ortho-chlor-para-trifluor-methylphenoxy)-benzoesäure, deren Herstellung, sie als Wirkstoff enthaltende herbizide Mittel und deren Verwendung.**

(30) Priorität: **09.01.79 CH 168/79**

(43) Veröffentlichungstag der Anmeldung: **23.07.80 Patentblatt 80/15**

(45) Bekanntmachung des Hinweises auf die Patenterteilung: **18.05.83 Patentblatt 83/20**

(84) Benannte Vertragsstaaten: **BE CH DE FR GB IT NL**

(56) Entgegenhaltungen:
**DE-A-2 019 821**
**DE-A-2 311 638**

(73) Patentinhaber: **CIBA-GEIGY AG, Patentabteilung Postfach, CH-4002 Basel (CH)**

(72) Erfinder: **Dürr, Dieter, Dr., Brändelistalweg 16, CH-4103 Bottmingen (CH)**
Erfinder: **Rohr, Otto, Dr., Kilbertweg 19, CH-4106 Therwil (CH)**
Erfinder: **Böhner, Beat, Dr., Hügelweg 3, CH-4102 Binningen (CH)**

EP 0 013 660 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

# Aminoakylester der 2-Nitro-5-(ortho-chlor-para-trifluormethylphenoxy)-benzoesäure, deren Herstellung, sie als Wirkstoff enthaltende herbizide Mittel und deren Verwendung

Die vorliegende Erfindung betrifft neue Aminoalkylester der 2-Nitro-5-(ortho-chlor-para-trifluormethylphenoxy)-benzoesäure mit herbizider Wirkung und deren Säureadditions- und quaternäre Ammoniumsalze, Verfahren zu deren Herstellung, sie als Wirkstoff enthaltende Mittel sowie deren Verwendung als selektive Herbizide, besonders in Kulturen von Soja und Reis im Nachanflaufverfahren .

Die Aminoalkylester der 2-Nitro-5-(ortho-chlor-para-trifluormethylphenoxy)-benzoesäure entsprechen der Forme I,

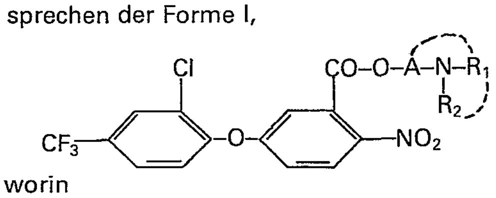 (I)

worin

A ein verzweigtes oder unverzweigtes $C_2-C_4$ Alkylenrest,

$R_1$ $C_1-C_4$ Alkyl, gegebenenfalls substituiert durch Halogen, Cyano oder Hydroxy oder durch Sauerstoff oder Schwefel unterbrochen, $C_3-C_5$ Alkenyl oder Alkinyl, gegebenenfalls durch Halogen substituiert, und

$R_2$ Wasserstoff oder dasselbe wie $R_1$ ist, oder

$R_1$ und $R_2$ zusammen mit dem Stickstoffatom, an das sie gebunden sind, in der Bedeutung von 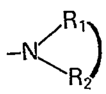, oder ein Teil des Alkylenrestes

A und $R_1$ zusammen mit dem Stickstoffatom, an das sie gebunden sind, in der Bedeutung von $-A-\underset{R_2}{\underset{|}{N}}-R_1$ einen am Stickstoffatom durch $R_2$ substituierten Pyrrolidin-, Piperidin-, Azepin-, Morpholin-, Thiomorpholin- oder Piperazinrest, oder ein Teil des Alkylenrestes A, $R_1$ und $R_2$ zusammen mit dem Stickstoffatom, an das sie gebunden sind, einen Pyridinrest bedeuten, wobei die heterocyclischen Reste durch $C_1-C_4$ Alkyl und der Piperazinring am zweiten Ringstickstoffatom auch durch $C_1-C_4$ Alkyl, Phenyl oder Benzyl substituiert sein können.

Als Wirkstoffe kommen ebenfalls die Säureadditions- und quaternären Ammoniumsalze dieser Aminoalkylester in Frage, welche der Formel Ia entsprechen

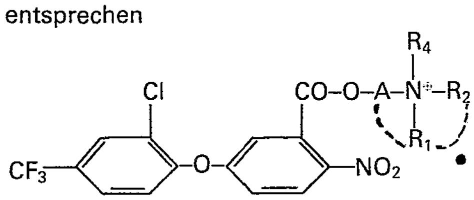 (Ia),

worin A, $R_1$ und $R_2$ die oben angegebene Bedeutung haben, $R_4$ $C_1-C_6$ Alkyl, gegebenenfalls substituiert durch Hydroxyl oder Methoxy; oder Benzyl und $B^-$ das Anion einer organischen oder anorganischen Säure bedeuten.

In dieser Formel umfassen die Alkylenreste A den Äthylen-, Propylen- und Butylenrest, wie die verzweigten Reste 1-Methyläthylen-, 2-Methyläthylen-, 1-Methylpropylen, 2-Methylpropylen-, 3-Methylpropylen sowie 1,2-Dimethyläthylen. Die für $R_1$, $R_2$ und $R_4$ sowie das zweite Ringstickstoffatom des Piperazinringes genannten Alkylreste können verzweigt oder unverzweigt sein, ebenso die Alkenyl- und Alkinylreste $R_1$ und $R_2$. Als Substituenten der Reste $R_1$ und $R_2$ kommen als Halogenatome besonders Chlor, Brom oder Jod in Frage, sie können ausserdem durch Cyan oder Hydroxyl substituiert oder durch Sauerstoff oder Schwefel unterbrochen sein.

Als zur Salzbildung geeignete Säuren kommen die Halogenwasserstoffe, die Schwefelsäure, organische Schwefelsäuren, Phosphorsäure oder organische Säuren, z. B. Fettsäuren in Betracht. Zur Quaternisierung eignen sich vor allem Alkyl- oder Benzylhalogenide sowie Alkyl- oder Benzylester der Toluolsulfonsäure.

Aus dem US-Patent 3928416 sowie der DE-A-2311638 sind Derivate der 2-Nitro-5-(ortho-chlor-para-trifluormethylphenoxy)-benzoesäure bekannt. Diese haben herbizide Wirkung, sind aber ziemlich phytotoxisch. Die erfindungsgemässen Aminoalkylester und deren Säureadditionssalze zeigen überraschenderweise bei gleich guter herbizider Wirkung eine wertvolle Selektivität gegenüber dikotylen Kulturen, vor allem gegenüber Soja, welche die bekannten Verbindungen nicht besitzen.

Im US Patent Nr. 3784635 sind ferner 2-Nitro-5-toluoloxy-benzoesäuren-ester und -amide mit herbizider Wirkung beschrieben.

Die Herstellung der Aminoalkylester der 2-Nitro-5-(ortho-chlor-para-trifluormethylphenoxy)-benzoesäure der Formel I und deren Säureadditionssalze geschieht in an sich bekannter Weise.

Nach einem ersten Verfahren setzt man ein Halogenid der 2-Nitro-5-(ortho-chlor-para-trifluormethylphenoxy)-benzoesäure der Formel II

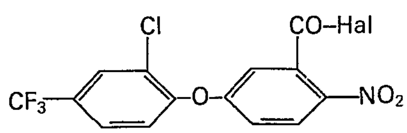 (II)

worin «Hal» Chlor oder Brom bedeutet, mit dem Aminoalkanol der Formel III um

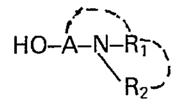 (III)

worin A, $R_1$ und $R_2$ die unter Formel I angegebene Bedeutung haben.

Die Umsetzung erfolgt vorteilhafterweise in einem den Reaktionspartnern gegenüber inerten Lösungsmittel, gegebenenfalls in Anwesenheit der molaren Menge eines säurebindenden Mittels, je nachdem man als Endprodukt den Aminoalkylester oder ein Säureadditionssalz davon haben will. Die Temperatur dieser Umsetzung kann zwischen der Raumtemperatur und dem Siedepunkt des Reaktionsgemisches variieren.

Einige Verbindungen kann man herstellen, indem man das Benzoesäurehalogenid der Formel II zuerst mit einem Halogenalkanol der Formel IV

HO–A–Hal (IV)

worin A die unter Formel I gegebene Bedeutung hat und Hal für Chlor oder Brom steht, zum Halogenalkylester der 2-Nitro-5-(ortho-chlor-para-trifluormethyl-phenoxy)-benzoesäure der Formel V

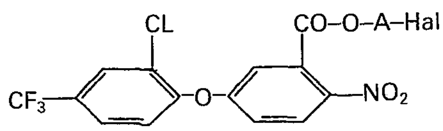

(V)

worin A und Hal die oben gegebenen Bedeutungen haben, und diesen dann mit einem Amin der Formel VI

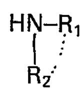

worin $R_1$ und $R_2$ die unter Formel I gegebene Bedeutung haben, umsetzt, gegebenenfalls in Anwesenheit der äquimolaren Menge eines säurebindenden Mittels. Auch diese Umsetzung wird vorteilhafterweise in einem den Reaktionsteilnehmern gegenüber inerten Lösungsmittel, bei einer Temperatur, die zwischen der Raumtemperatur und dem Siedepunkt des Reaktionsgemisches liegt, vorgenommen.

Falls sie nicht bereits in Salzform aus der Reaktion anfallen, können Säureadditionssalze der Aminoalkylester der Formel I in einfacher Weise durch Zusammengeben äquimolarer Mengen des Aminoesters und der Säure in einem inerten Lösungsmittel erhalten werden.

Umgekehrt kann aus einem Säureadditionssalz der freie Aminoester gewonnen werden durch Zugeben eines kleinen Überschusses einer Base, am besten eines tertiären Amins, in wässriger Lösung in Gegenwart eines inerten, mit Wasser nicht mischbaren Lösungsmittels. Der Ester geht in die Lösungsmittelphase und kann durch Verdampfen des Lösungsmittels erhalten werden.

Die quaternären Ammoniumsalze können ebenfalls durch Zusammengeben äquimolarer Mengen des Aminoesters der Formel I mit einem Ester der Formel VI,

$R_4$–B (VI)

worin B und $R_4$ die gegebene Bedeutung haben, in einem inerten Lösungsmittel erhalten und nach Verdampfen des Lösungsmittels isoliert werden.

Die Herstellung der 2-Nitro-5-(ortho-chlor-para-trifluormethylphenoxy)-benzoesäure ist bekannt und im US Patent Nr. 3928416 beschrieben.

Die Herstellung der erfindungsgemässen Aminoalkylester kann aus den nachfolgenden Beispielen ersehen werden. Die Temperaturen sind darin in Celsiusgraden gegeben, Prozentangaben und Teile beziehen sich auf das Gewicht. Im Anschluss an die Beispiele sind in einer Tabelle weitere in analoger Weise hergestellte Verbindungen aufgeführt.

Die erfindungsgemässen Aminoalkylester sind für Menschen und Tiere wenig giftig, ihre Handhabung bedarf keiner besonderen Vorsichtsmassnahmen. Sie sind in den gebräuchlichen Lösungsmitteln wie Kohlenwasserstoffen, Äthern, Ketonen und chlorierten Kohlenwasserstoffen löslich.

Beispiel 1

2-Nitro-5-(ortho-chlor-para-trifluormethylphenoxy)-benzoesäure-2″-dimethylaminoäthyl-ester

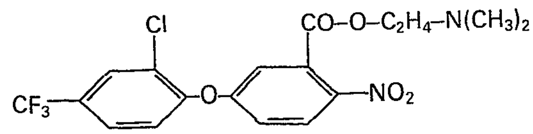

18,5 g 2-Nitro-5-(ortho-chlor-para-trifluormethylphenoxy)-benzoesäure werden in 100 ml Toluol mit 10 ml Thionylchlorid und einem Tropfen Dimethylformamid zwei Stunden auf 80° erhitzt, dann wird das Lösungsmittel am Vakuum abdestilliert und der Rückstand in 50 ml Toluol aufgenommen. Diese Lösung von 2-Nitro-5-(ortho-chlor-para-trifluormethylphenoxy)-benzoylchlorid wird bei 20–60° unter Rühren zu einer Lösung von 20 g 2-Dimethylaminoäthanol und 28 ml Triäthylamin in 200 ml Toluol getropft. Nach einer Stunde giesst man das Reaktionsgemisch auf Wasser. Die organische Schicht wird mit Wasser gewaschen, getrocknet und eingedampft. Man erhält in fast quantitativer Ausbeute den oben genannten Ester als Öl, das nach dem Anreiben mit Hexan erstarrt, Schmelzpunkt 73–75°.

Beispiel 2

2-Nitro-5-(ortho-chlor-para-trifluormethylphenoxy)-benzoesäure-2″-methylaminoäthyl-ester. Hydrochlorid

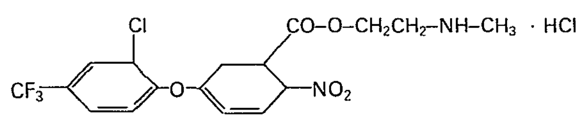

20 g 2-Nitro-5-(ortho-chlor-para-trifluormethylphenoxy)-benzoesäure-2″-bromäthylester werden mit 2,8 g Methylamin in 300 ml Äther stehen gelassen. Der Reaktionsverlauf wird mit Dünnschichtchromatographie verfolgt. Zur Aufarbeitung wird das Reaktionsgemisch mit kalter verdünnter Sodalösung verrührt, die Ätherlösung wird filtriert, durch Eindampfen erhält man die freie Base der Titelverbindung als dickflüssiges Öl. Das Hydrochlorid wird durch Einleiten von trockenem Salzsäuregas in die Ätherlösung bereitet. Schmelzpunkt 105°.

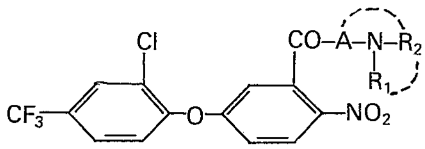

| Nr. | 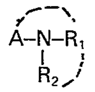 | Physikalische Konstante |
|---|---|---|
| 1 | $-C_2H_4N(CH_3)_2$ | Smp. 73–75° |
| 2 | $-C_3H_6N(CH_3)_2$ | $n_D^{23.5}$ 1.5314 |
| 3 | $CH(CH_3)CH_2N(CH_3)_2$ | $n_D^{24.5}$ 1.5285 |
| 4 | 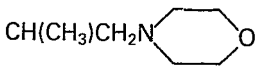 | $n_D^{22}$ 1.5296 |
| 5 | $-C_2H_4N(C_2H_5)_2$ | $n_D^{24}$ 1.5350 |
| 6 | $-CH(CH_3)CH_2N(CH_3)_2$ | $n_D^{24}$ 1.5394 |
| 7 | 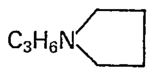 | |
| 8 | 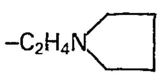 | $n_D^{23}$ 1.5455 |
| 9 | 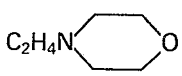 | $n_D^{25}$ 1.5455 |
| 10 | 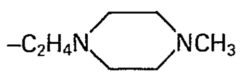 | |
| 11 | $-C_2H_4N(CH_2CH=CH_2)_2$ | |
| 12 | $C_2H_4N(CH_2C\equiv CH)_2$ | |
| 13 | $C_2H_4N(C_2H_4OH)_2$ | |
| 14 | $-C_2H_4N(CH_3)-C_2H_4OCH_3$ | |
| 15 | $-C_4H_8N(CH_3)_2$ | |
| 16 | $-C_2H_4N^{\oplus}H_2CH_3\ Cl^{\ominus}$ | Smp. 105° |
| 17 | $-C_2H_4N(CH_3)C_2H_5$ | $n_D^{24}$ 1.5395 |
| 18 | 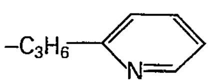 | Smp. 72–75° |
| 19 | 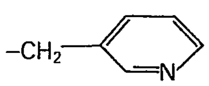 | $n_D^{23}$ 1.5268 |
| 20 | 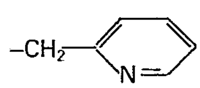 | $n_D^{25}$ 1.5655 |
| 21 | 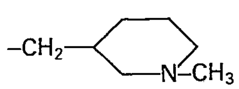 | $n_D^{23}$ 1.5460 |
| 22 | 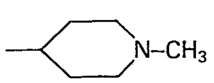 | $n_D^{23}$ 1.5501 |
| 23 | $-C_2H_4N^{\oplus}(CH_3)_3\ I^{\ominus}$ | Smp. 176–9° |
| 24 | 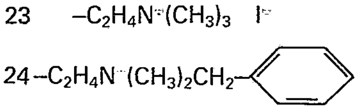 $Cl^{\ominus}$ | Smp. 143–6° |
| 25 | 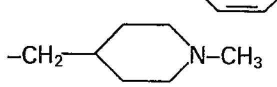 | |

Erfindungsgemässe Mittel, die als aktive Komponente mindestens eine Verbindung der Formel I enthalten, eignen sich besonders zur selektiven Bekämpfung schwerbekämpfbarer Ungräser in pre- und insbesondere post-emergenter Anwendung in Kulturpflanzenbeständen, wie Soja, Baumwolle.

Zum Nachweis der Brauchbarkeit als Herbizide (pre- und post-emergent) dienen folgende Testmethoden:

Pre-emergente Herbizid-Wirkung
(Keimhemmung)

Im Gewächshaus wird unmittelbar nach der Einsaat der Versuchspflanzen in Saatschalen die Erdoberfläche mit einer wässerigen Dispersion der Wirkstoffe, erhalten aus einem 25%igen Emulsionskonzentrat resp. aus einem 27%igen Spritzpulver mit Wirkstoffen, die wegen ungenügender Löslichkeit nicht als Emulsionskonzentrat hergestellt werden können, behandelt. Es wurden vier verschiedene Konzentrationsreihen angewendet, entsprechend 4, 2, 1 und 0,5 kg Wirksubstanz pro Hektar. Die Saatschalen werden im Gewächshaus bei 22–25°C und 50–70% rel. Luftfeuchtigkeit gehalten und der Versuch nach 3 Wochen ausgewertet und die Resultate nach folgender Notenskala bonitiert:

1 = Pflanzen nicht gekeimt oder total abgestorben
2–3 = sehr starke Wirkung
4–6 = mittlere Wirkung
7–8 = geringe Wirkung
9 = keine Wirkung (wie unbehandelte Kontrolle)
– = Pflanze in dieser Wirkstoffkonzentration nicht geprüft

Post-emergente Herbizid-Wirkung
(Kontaktherbizid)

In diesem Versuch ist die Verbindung Nr. 1 mit der 2-Nitro-5-(ortho-chlor-para-trifluormethylphenoxy)-benzoesäure, bekannt aus dem US Patent Nr. 3928416, Beispiel 48 = Verbindung A verglichen worden. Die Resultate sind in der untenstehenden Tabelle zusammengefasst worden.

Eine grössere Anzahl (mindestens 7) Unkräuter und Kulturpflanzen, sowohl monocotyle wie dicotyle, wurde nach dem Anlaufen (im 4- bis 6-Blattstadium) mit einer wässrigen Wirkstoffdispersion in Dosierungen von 0,06; 0,125; 0,25; 0,5; 1; 2 und 4 kg Wirksubstanz pro Hektar auf die Pflanzen gespritzt und diese bei 24–26°C und 45–60% rel. Luftfeuchtigkeit gehalten. 15 Tage nach Behandlung wird der Versuch ausgewertet und das Ergebnis wie im pre-emergent-Versuch nach derselben Notenskala bonitiert.

| Geprüfte Verbindung | Nr. 1 | | | | A | | | |
|---|---|---|---|---|---|---|---|---|
| Aufwandmenge in kg/ha | 4 | 2 | 1 | ½ | 4 | 2 | 1 | ½ |
| Pflanze | | | | | | | | |
| Soja | 8 | 8 | 9 | 9 | 3 | 4 | 5 | 5 |
| Weizen | 3 | 7 | 8 | 9 | 2 | 4 | 4 | 8 |
| Mais | 3 | 5 | 9 | 9 | 1 | 2 | 3 | 3 |
| abutilon sp. | 1 | 1 | 1 | 2 | 1 | 1 | 1 | 2 |
| sesbania exaltata | 1 | 1 | 1 | 2 | 1 | 1 | 1 | 1 |
| amaranthus retroflexus | 1 | 1 | 1 | 1 | 1 | 1 | 1 | 1 |
| ipomoea purpurea | 1 | 1 | 1 | 2 | 1 | 1 | 1 | 1 |

Die neuen Wirkstoffe der Formel I sind stabile Verbindungen, welche in üblichen organischen Lösungsmitteln, wie Alkoholen, Äthern, Ketonen, Dimethylformamid und Dimethylsulfoxyd, löslich sind.

Die Herstellung erfindungsgemässer Mittel erfolgt in an sich bekannter Weise durch inniges Vermischen und Vermahlen von Wirkstoffen der allgemeinen Formel I mit geeigneten Trägerstoffen und/oder Verteilungsmitteln, gegebenenfalls unter Zusatz von gegenüber den Wirkstoffen inerten Antischaum-, Netz-, Dispersions- und/oder Lösungsmitteln. Die Wirkstoffe können in den folgenden Aufarbeitungsformen vorliegen und angewendet werden:

Feste Aufarbeitungsformen:
Stäubemittel, Streumittel, Granulate, Umhüllungsgranulate, Imprägnierungsgranulate und Homogengranulate.

In Wasser dispergierbare Wirkstoffkonzentrate:
Spritzpulver (wettable powder), Paste, Emulsionen; Emulsionskonzentrate.

Flüssige Aufarbeitungsformen:
Lösungen.

Die Wirkstoffkonzentrationen betragen in den erfindungsgemässen Mitteln 1 bis 80 Gew.-% und können gegebenenfalls bei der Anwendung auch in geringen Konzentrationen wie etwa 0,05 bis 1% vorliegen.

Den beschriebenen erfindungsgemässen Mitteln lassen sich andere biozide Wirkstoffe oder Mittel beimischen.

Die folgenden Formulierungsbeispiele sollen die Herstellung von festen und flüssigen Aufbereitungsformen näher erläutern.

Emulsionskonzentrat

Zur Herstellung eines 25%igen Emulsionskonzentrates werden

25 Teile eines Wirkstoffes der Formel I,
5 Teile einer Mischung von Nonylphenolpolyoxyäthylen oder Calciumdodecylbenzolsulfat,
15 Teile Cyclohexanon,
55 Teile Xylol

miteinander vermischt. Dieses Konzentrat kann mit Wasser zu Emulsionen auf geeigneten Konzentrationen von z. B. 0,1 bis 10% verdünnt werden. Solche Emulsionen eignen sich zur Bekämpfung von Unkräutern in Kulturpflanzungen.

Granulate

Zur Herstellung eines 5%igen Granulates werden die folgenden Stoffe verwendet:

5 Teile eines der Wirkstoffe der Formel I,
0,25 Teile Epichlorhydrin,
0,25 Teile Cetylpolyglykoläther,
3,50 Teile Polyäthylenglykol,
91 Teile Kaolin (Korngrösse: 0,3–0,8 mm).

Die Aktivsubstanz wird mit Epichlorhydrin vermischt und in 6 Teilen Aceton gelöst, hierauf wird Polyäthylenglykol und Cetylpolyglykoläther zugesetzt. Die so erhaltene Lösung wird auf Kaolin aufgesprüht und anschliessend im Vakuum verdampft.

Spritzpulver

Zur Herstellung eines a) 70%igen und b) 10%igen Spritzpulvers werden folgende Bestandteile verwendet:

a) 70 Teile eines Wirkstoffes der Formel I,
5 Teile Natriumdibutylnaphthylsulfonat,
3 Teile Naphthalinsulfonsäuren-Phenolsulfonsäuren-Formaldehyd-Kondensat 3:2:1,
10 Teile Kaolin,
12 Teile Champagne-Kreide;

b) 10 Teile eines Wirkstoffes der Formel I,
3 Teile Gemisch der Natriumsalze von gesättigten Fettalkoholsulfaten,
5 Teile Naphthalinsulfonsäuren-Formaldehyd-Kondensat,
82 Teile Kaolin.

Der angegebene Wirkstoff wird auf die entsprechenden Trägerstoffe (Kaolin und Kreide) aufgezogen und anschliessend mit den übrigen Bestandteilen vermischt und vermahlen. Man erhält Spritzpulver von vorzüglicher Benetzbarkeit und Schwebefähigkeit. Aus solchen Spritzpulvern können durch Verdünnen mit Wasser Suspensionen von 0,1–80% Wirkstoff erhalten werden, die sich zur Unkrautbekämpfung in Pflanzenkulturen eignen.

Paste

Zur Herstellung einer 45%igen Paste werden folgende Stoffe verwendet:

45 Teile eines Wirkstoffes der Formel I,
5 Teile Natriumaluminiumsilikat,
14 Teile Cetylpolyglykoläther mit 8 Mol Äthylenoxid,
1 Teil Oleylpolyglykoläther mit 5 Mol Äthylenoxid,
2 Teile Spindelöl,
10 Teile Polyäthynglykol,
23 Teile Wasser.

Der Wirkstoff wird mit den Zuschlagstoffen in dazu geeigneten Geräten innig vermischt und vermahlen. Man erhält eine Paste, aus der sich durch Verdünnen mit Wasser Suspensionen jeder gewünschten Konzentration herstellen lassen.

**Patentansprüche**

1. Aminoalkylester der 2-Nitro-5-(ortho-chlor-para-trifluormethylphenoxy)-benzoesäure der Formel I und ihre Säureadditionssalze

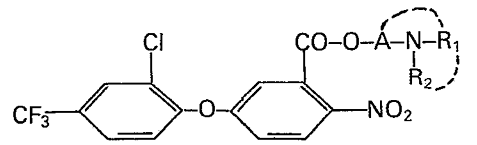 (I)

worin

A ein verzweigter oder unverzweigter $C_2-C_4$-Alkylenrest,

$R_1$ $C_1-C_4$ Alkyl, gegebenenfalls substituiert durch Halogen, Cyano oder Hydroxyl oder durch Sauerstoff oder Schwefel unterbrochen, $C_3-C_5$ Alkenyl oder Alkinyl, gegebenenfalls durch Halogen substituiert und

$R_2$ Wasserstoff oder dasselbe wie $R_1$ ist, oder

$R_1$ und $R_2$ zusammen mit dem Stickstoffatom, an das sie gebunden sind, in der Bedeutung von 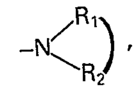, oder ein Teil des Alkylenrestes

A und $R_1$ zusammen mit dem Stickstoffatom, an das sie gebunden sind, in der Bedeutung von 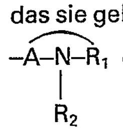 einen am Stickstoffatom durch $R_2$ substituierten Pyrrolidin-, Piperidin-, Azepin-, Morpholin-, Thiomorpholin- oder Piperazinrest, oder ein Teil des Alkylenrestes

A, $R_1$ und $R_2$ zusammen mit dem Stickstoffatom, an das sie gebunden sind, einen Pyridinrest bedeuten, wobei die heterocyclischen Reste durch $C_1-C_4$ Alkyl und der Piperazinring am zweiten Ringstickstoffatom auch durch $C_1-C_4$ Alkyl, Phenyl oder Benzyl substituiert sein können.

2. Die Säureadditions- und quaternären Ammoniumsalze der Aminoalkylester gemäss Anspruch 1 entsprechend der Formel Ia,

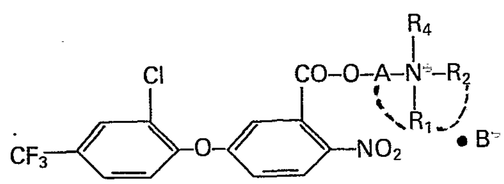 (Ia),

worin A, $R_1$ und $R_2$ die in Formel I, Anspruch 1 gegebene Bedeutung haben, $R_4$ $C_1-C_6$ Alkyl, gegebenenfalls substituiert durch Hydroxyl oder Methoxy; oder Benzyl und $B^{\ominus}$ das Anion einer organischen oder anorganischen Säure bedeutet.

3. Als Verbindung der Formel I gemäss Anspruch 1, 2-Nitro-5-(ortho-chlor-para-trifluormethylphenoxy)-benzoesäure-2'-dimethylamino-äthylester.

4. Als Verbindung der Formel I gemäss Anspruch 1, 2-Nitro-5-(ortho-chlor-para-trifluormethylphenoxy)-benzoesäure-1'-morpholinoprop-2'-yl-ester.

5. Als Verbindung der Formel I gemäss Anspruch 1, 2-Nitro-5-(ortho-chlor-para-trifluormethylphenoxy)-benzoesäure-1'-diäthylamino-prop-2'-yl-ester.

6. Als Verbindung der Formel I gemäss Anspruch 1, 2-Nitro-5-(ortho-chlor-para-trifluormethylphenoxy)-benzoesäure-pyrid-2'-yl-methylester.

7. Als Verbindung der Formel I gemäss Anspruch 1, 2-Nitro-5-(ortho-chlor-para-trifluormethylphenoxy)-benzoesäure-(3'-pyrid-2"-yl-propyl)-ester.

8. Die Aminoalkylester und Säureadditionssalze derselben der Formel I, Anspruch 1, in denen A einen verzweigten oder unverzweigten $C_2-C_4$ Alkylenrest bedeutet.

9. Verfahren zur Herstellung von Aminoalkylester der Formel I gemäss Anspruch 1, dadurch gekennzeichnet, dass man ein Halogenid der 2-Nitro-5-(ortho-chlor-para-trifluormethylphenoxy)-benzoesäure der Formel II

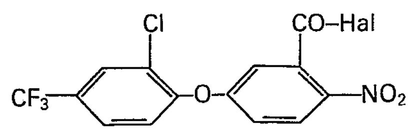 (II)

worin Hal Chlor oder Brom bedeutet, mit einem Aminoalkanol der Formel III umsetzt,

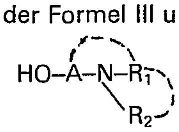 (III)

worin A, $R_1$ und $R_2$ die unter Formel I im Anspruch 1 gegebene Bedeutung haben, und gegebenenfalls den erhaltenen Aminoalkylester mit einer Säure in ein Säureadditionssalz umsetzt.

10. Verfahren zur Herstellung der Aminoalkylester der Formel I gemäss Anspruch 1, dadurch gekennzeichnet, dass man einen Halogenalkylester der 2-Nitro-5-(ortho-chlor-para-trifluormethylphenoxy)-benzoesäure der Formel V

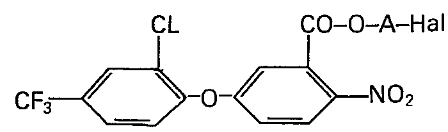 (V)

worin A die unter Formel I im Anspruch 1 gegebene Bedeutung hat und Hal Chlor oder Brom bedeutet, mit einem Amin der Formel IV

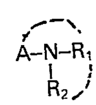 (IV)

umsetzt, worin $R_1$ und $R_2$ die unter Formel I im Anspruch 1 gegebene Bedeutung haben, und gegebenenfalls den erhaltenen Aminoester mit einer Säure in ein Säureadditionssalz umsetzt.

11. Verfahren zur Herstellung der quaternären Ammoniumsalze der Aminoester der Formel Ia gemäss Anspruch 2, dadurch gekennzeichnet, dass man einen Aminoester der Formel I gemäss Anspruch 1 in einem inerten Lösungsmittel mit der äquimolaren Menge eines Esters der Formel VI

$$R_4{-}B \qquad \text{(VI)}$$

worin B ein Halogenatom oder das Anion einer organischen oder anorganischen Säure und $R_4$ $C_1-C_6$ Alkyl, gegebenenfalls substituiert durch Hydroxyl oder Methoxy; oder Benzyl bedeutet, umsetzt, und das Lösungsmittel nachher verdampft.

12. Herbizides Mittel, dadurch gekennzeichnet, dass es neben inerten Zutaten, als Wirkstoff einen Aminoalkylester der Formel I oder ein Salz davon gemäss Ansprüchen 1 und 2 enthält.

13. Die Verwendung der Aminoalkylester der Formel I gemäss Anspruch 1, ihrer Salze gemäss Ansprüchen 1 und 2 oder sie enthaltender Mittel gemäss Anspruch 12 als selektive Herbizide.

14. Die Verwendung der Aminoalkylester der Formel I gemäss Anspruch 1, ihrer Salze gemäss Ansprüchen 1 und 2 oder sie enthaltender Mittel, gemäss Anspruch 12 zur selektiven Bekämpfung von Unkräutern in Kulturen von Soja und Reis im Nachauflaufverfahren.

**Claims**

1. An aminoalkylester of 2-nitro-5-(ortho-chloro-para-trifluormethylphenoxy)-benzoic acid of the formula I and acid addition salts thereof

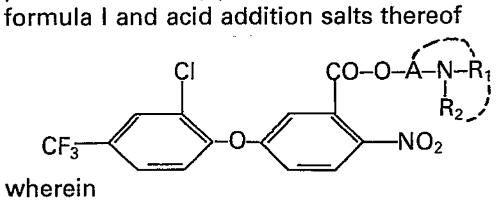
(I)

wherein

A is a branched-chain or straight-chain $C_2-C_4$-alkylene group,

$R_1$ is $C_1-C_4$-alkyl which is unsubstituted or substituted by halogen, cyano or hydroxyl, or is interrupted by oxygen or sulfur, or it is $C_3-C_5$-alkenyl or alkynyl, both unsubstituted or substituted by halogen,

$R_2$ is hydrogen or has the same meaning as $R_1$, or

$R_1$ and $R_2$ together with the nitrogen atom to which they are bound as 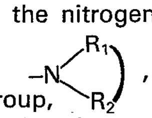, or a portion of the alkylene group,

A and $R_1$ together with the nitrogen atom to which they are bound as 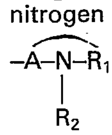, form a pyrrolidine, piperidine, azepine, morpholine, thiomorpholine or piperazine group, each unsubstituted or substituted on the nitrogen atom by $R_2$, or a portion of the alkylene group

A, $R_1$ and $R_2$ together with the nitrogen atom to which they are bound form a pyridine group, whereby the heterocyclic radicals can be substituted by $C_1-C_4$-alkyl, and the piperazine ring on the second ring nitrogen atom also by $C_1-C_4$-alkyl, phenyl or benzyl.

2. The acid addition salts and quaternary ammonium salts of the aminoalkyl ester according to claim 1 which correspond to the formula Ia

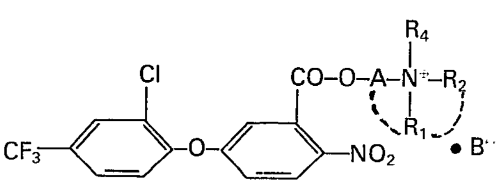
(Ia),

wherein A, $R_1$ and $R_2$ have the meanings defined under the formula I in claim 1, $R_4$ is $C_1-C_6$-alkyl which is unsubstituted or is substituted by hydroxyl or methoxy, or it is benzyl, and B is the anion of an organic or inorganic acid.

3. As compound of the formula I according to claim 1, 2-nitro-5-(o-chloro-p-trifluormethyl-phenoxy-benzoic acid-2'-dimethylaminoethyl ester.

4. As compound of the formula I according to claim 1, 2-nitro-5-(o-chloro-p-trifluormethyl-phenoxy)-benzoic acid-1'-morpholinoprop-2'-yl ester.

5. As compound of the formula I according to claim 1, 2-nitro-5-(o-chloro-p-trifluormethyl-phenoxy)-benzoic acid-1'-diethylamino-prop-2'-yl ester.

6. As compound of the formula I according to claim 1, 2-nitro-5-(o-chloro-p-trifluoromethyl-phenoxy)-benzoic acid-pyrid-2'-yl-methyl ester.

7. As compound of the formula I according to claim 1, 2-nitro-5-(o-chloro-p-trifluoromethyl-phenoxy)-benzoic acid-(3'-pyrid-2''-yl-propyl) ester.

8. An aminoalkyl ester, and acid addition salts thereof, of the formula I according to claim 1, wherein A is a branched-chain or straight-chain $C_2-C_4$-alkylene group.

9. A process for producing an aminoalkyl ester of the formula I according to claim 1, which process comprises reacting a halide of 2-nitro-5-(o-chloro-p-trifluoromethylphenoxy)-benzoic acid of the formula II

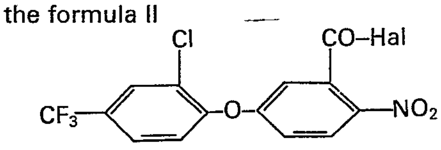
(II)

wherein «Hal» is chlorine or bromine, with an aminoalkanol of the formula III

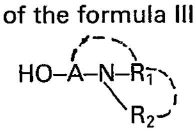
(III)

wherein A, $R_1$ and $R_2$ have the meanings defined under the formula I in claim 1, and optionally converting the resulting aminoalkyl ester with an acid into an acid addition salt.

10. A process for producing an aminoalkyl ester of the formula I according to claim 1, which process comprises reacting a halogenoalkyl ester of 2-nitro-5-(o-chloro-p-trifluoromethylphenoxy)-benzoic acid of the formula V

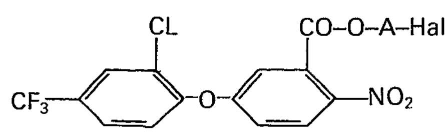
(V)

wherein A has the meaning defined under the formula I in claim 1, and «Hal» is chlorine or bromine, with an amine of the formula IV

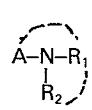
(IV)

wherein $R_1$ and $R_2$ have the meanings defined under the formula I in claim 1, and optionally converting the resulting amino ester with an acid into an acid addition salt.

11. A process for producing the quaternary ammonium salts of the amino ester of the formula Ia according to claim 2, which process comprises reacting an amino ester of the formula I according to claim 1, in an inert solvent, with the equimolar amount of an ester of the formula VI

$$R_4\text{–}B \qquad (VI)$$

wherein B is a halogen atom or the anion of an organic or inorganic acid, and $R_4$ is $C_1-C_6$-alkyl which is unsubstituted or substituted by hydroxyl or methoxy, or $R_4$ is benzyl, and subsequently evaporating off the solvent.

12. A herbicidal composition containing as active substance an aminoalkyl ester of the formula I or a salt thereof according to claims 1 and 2, together with inert ingredients.

13. The use of an aminoalkyl ester of the formula I according to claim 1, or of salts thereof according to claims 1 and 2, or of compositions according to claim 12 containing the said ester or salts thereof, as selective herbicides.

14. The use of an aminoalkyl ester of the formula I according to claim 1, or of salts thereof according to claim 2, or of compositions according to claim 12 containing the said ester or salts thereof, for selectively controlling weeds in cultivated crops of soya bean and rice using the post-emergence process.

**Revendications**

1. Esters aminoalkyliques de l'acide 2-nitro-5-(ortho-chloro-para-trifluorométhylphénoxy)-benzoïque de formule I et leurs sels formés par addition avec des acides

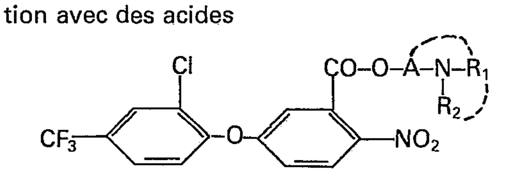

(I)

dans laquelle

A représente un reste alkylène ramifié ou non en C2–C4,

$R_1$ représente un groupe alkyle en C1–C4 éventuellement substitué par des halogènes, des groupes cyano ou hydroxy ou interrompu par l'oxygène ou le soufre, un groupe alcényle ou alcynyle en C3–C5 éventuellement substitué par des halogènes et

$R_2$ représente l'hydrogène ou a la même signification que $R_1$ ou bien

$R_1$ et $R_2$ forment ensemble et avec l'atome d'azote sur lequel ils sont fixés, dans la signification de 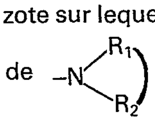 ou une partie du reste alkylène,

A et $R_1$ forment ensemble et avec l'atome sur lequel ils sont fixés, dans la signification de 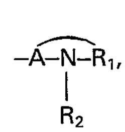, un reste de pyrrolidine, de pipéridine, d'azépine, de morpholine, de thiomorpholine ou de pipérazine éventuellement substitué à l'atome d'azote par $R_2$, ou une partie du reste alkylène,

A, $R_1$ et $R_2$ forment ensemble et avec l'atome d'azote sur lequel ils sont fixés un reste de pyridine, les restes hétérocycliques pouvant être substitués par des groupes alkyles en C1–C4 et le cycle pipérazine par des groupes alkyles en C1–C4, phényle ou benzyle sur le deuxième atome d'azote cyclique.

2. Les sels formés par addition avec des acides et sels d'ammonium quaternaire des esters aminoalkyliques selon la revendication 1, répondant à la formule Ia

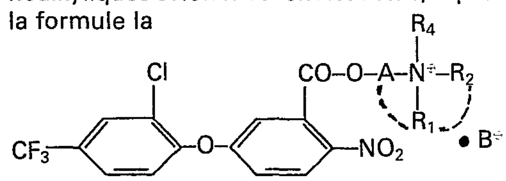

(Ia),

dans laquelle A, $R_1$ et $R_2$ ont les significations indiquées en référence à la formule I, revendication 1, $R_4$ représente un groupe alkyle en C1–C6 éventuellement substitué par des groupes hydroxy ou méthoxy; ou un groupe benzyle, et B est l'anion d'un acide organique ou minéral.

3. En tant que composé de formule I, revendication 1, l'ester 2'-diméthylaminoéthylique de l'acide 2-nitro-5-(ortho-chloro-para-trifluorométhylphénoxy)-benzoïque.

4. En tant que composé de formule I selon la revendication 1, l'ester 1'-morpholinopropa-2'-ylique de l'acide 2-nitro-5-(ortho-chloro-para-trifluorométhylphénoxy)-benzoïque.

5. En tant que composé de formule I selon la revendication 1, l'ester 1'-diéthylamino-propa-2'-ylique de l'acide 2-nitro-5-(ortho-chloro-para-trifluorométhylphénoxy)benzoïque.

6. En tant que composé de formule I selon la revendication 1, l'ester pyrido-2'-yl-méthylique de l'acide 2-nitro-5-(ortho-chloro-para-trifluorométhylphénoxy)-benzoïque.

7. En tant que composé de formule I selon la revendication 1, l'ester 3'-pyrido-2''-yl-propylique de l'acide 2-nitro-5-(ortho-chloro-para-trifluorométhylphénoxy)-benzoïque.

8. Les esters aminoalkyliques et leurs sels formés par addition avec des acides de formule I selon la revendication 1 dans lesquels A représente un reste alkylène ramifié ou non en C2–C4.

9. Procédé de préparation des esters aminoalkyliques de formule I selon la revendication 1, caractérisé en ce que l'ont fait réagir un halogénure de l'acide 2-nitro-5-(ortho-chloro-para-trifluorométhylphénoxy)-benzoïque de formule II

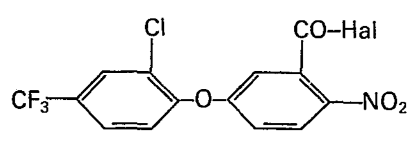

(II)

dans laquelle Hal représente le chlore ou le brome, avec un aminoalcanol de formule III

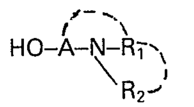

(III)

dans laquelle A, $R_1$ et $R_2$ ont les significations indiquées en référence à la formule I dans la revendication 1, et le cas échéant on convertit l'ester aminoalkylique obtenu en sel formé par addition avec un acide par réaction avec un acide.

10. Procédé de préparation des esters aminoalkyliques de formule I selon la revendication 1, caractérisé en ce que l'on fait réagir l'acide 2-nitro-5-(ortho-chloro-para-trifluorométhylphénoxy)-benzoïque de formule V

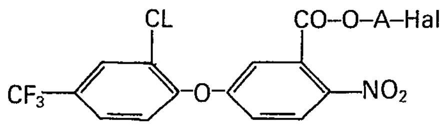

(V)

dans laquelle A a la signification indiquée en référence à la formule I dans la revendication 1 et Hal représente le chlore ou le brome, avec une amine de formule IV

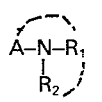

(IV)

dans laquelle $R_1$ et $R_2$ ont les significations indiquées en référence à la formule I dans la revendication 1, et le cas échéant on convertit l'aminoester obtenu en sel formé par addition avec un acide, par réaction avec un acide.

11. Procédé de préparation des sels d'ammonium quaternaire des aminoesters de formule Ia selon la revendication 2, carctérisé en ce que l'on fait réagir un aminoester de formule I selon la revendication 1 dans un solvant inerte avec la quantité équimoléculaire d'un ester de formule VI

$$R_4\text{–}B \qquad \text{(VI)}$$

dans laquelle B représente un atome d'halogène ou l'anion d'un acide organique ou minéral et $R_4$ représente un groupe alkyle en C1-C6 éventuellement substitué par des groupes hydroxy ou méthoxy; ou un groupe benzyle, et on élimine ensuite le solvant par évaporation.

12. Produit herbicide caractérisé en ce qu'il contient, avec des additifs inertes, un ester aminoalkylique de formule I ou un sel d'un tel ester selon les revendications 1 et 2 en tant que substance active.

13. L'utilisation des esters aminoalkyliques de formule I selon la revendication 1, de leurs sels selon les revendications 1 et 2 ou de produits en contenant selon la revendication 12 en tant qu'herbicides sélectifs.

14. Utilisation des esters aminoalkyliques de formule I selon la revendication 1, de leurs sels selon la revendication 2 ou de produits en contenant selon la revendication 12, pour la lutte sélective contre les mauvaises herbes dans les cultures de soja et de riz en application après sortie de terre.